(19) **Europäisches Patentamt / European Patent Office / Office européen des brevets**

(11) **EP 2 394 654 A1**

(12) **EUROPEAN PATENT APPLICATION**
published in accordance with Art. 153(4) EPC

(43) Date of publication:
**14.12.2011 Bulletin 2011/50**

(51) Int Cl.:
*A61K 36/53* (2006.01)     *A61K 9/06* (2006.01)
*A61K 9/08* (2006.01)      *A61K 9/14* (2006.01)
*A61K 9/48* (2006.01)      *A61P 1/16* (2006.01)
*A61P 13/12* (2006.01)     *A61P 29/00* (2006.01)
*A61P 37/00* (2006.01)     *A61P 37/02* (2006.01)
*A61K 125/00* (2006.01)

(21) Application number: **09839503.1**

(22) Date of filing: **06.02.2009**

(86) International application number:
**PCT/CN2009/000134**

(87) International publication number:
**WO 2010/088786 (12.08.2010 Gazette 2010/32)**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicants:
• **Lin, Xiuying**
**Anshun, Guizhou 561000 (CN)**
• **Mou, Linxiang**
**Anshun, Guizhou 561000 (CN)**

(72) Inventors:
• **Lin, Xiuying**
**Anshun, Guizhou 561000 (CN)**
• **Mou, Linxiang**
**Anshun, Guizhou 561000 (CN)**

(74) Representative: **Bergmeier, Werner**
**Canzler & Bergmeier**
**Friedrich-Ebert-Straße 84**
**85055 Ingolstadt (DE)**

(54) **HERBAL COMPOSITION HAVING COX-2 AND IMMUNE ACTIVITIES AND PREPARATION METHOD THEREOF**

(57)     Herbal composition having natural COX-2 and immune activities is disclosed, it contains a plant powder of Labiatae. The method for preparing the herbal composition comprises the steps of dehydrating a plant of labiatae and grinding it into a powder.

Fig. 6

EP 2 394 654 A1

**Description**

**Field of Invention**

The present invention relates to a herbal composition, and more particularly to a herbal composition having COX-2 and immune activities and its manufacturing method, which composition has notable effects in the treatment of acute and chronic nephritis, cholecystitis (including bile-reflux cholecystitis), pathological changes of lymphatic system like lymphatic tumors or submandibular gland tumors, gastric tumors, mastitis, and the recovery after polyp surgery of either throat or other human body parts.

**Description of Related Arts**

Cyclooxygenase-2 (COX-2 immune activity) was discovered in 1988. COX-2 immune activity is a protein acting as an enzyme to speed up the production of certain chemical messengers, and is especially effective during inflammation and pain. Accordingly, when COX-2 immune activity is inhibited, inflammation is reduced.

According to traditional Chinese (herbal) medicine theory, effective chemical components may be extracted from natural herbs. Therefore, if we successfully find out and extract that functional chemical from the herbs, the patients can effectively take the dose of concentrated functional chemical instead of having the whole plant of the herbs. The extracted concentrated chemical medicine is more effective and more convenient for use, jut like the Western medicine.

**SUMMARY OF THE PRESENT INVENTION**

A main object of the present invention is to provide a herbal composition having natural COX-2 and immune activities, as well as its manufacturing method, which composition has notable effects in curing acute and chronic nephritis, cholecystitis (including bile-reflux cholecystitis), pathological changes of lymphatic system like lymphatic tumors or submandibular gland tumors, gastric tumors, mastitis, and the recovery after polyp surgery of either throat or other human body parts.

Another object of the present invention is to provide a herbal composition having natural COX-2 and immune activity, and its manufacturing method, wherein the herbal composition is extracted from a new and distinct variety of herbal Labiatae plant, which is a grass-like plant given the varietal denomination of "Guizhou Qizhen Cao".

Another object of the present invention is to provide a herbal composition having natural COX-2 and immune activity, and its manufacturing method, which composition has an administrative form selected from the group consisting of capsule, powder, solution, and syrup.

Another object of the present invention is to provide a herbal composition having natural COX-2 and immune activity and its manufacturing method, which composition can be formed as an external treatment by mixing the powdered Labiatae plant with "Vaseline".

Another object of the present invention is to provide a herbal composition having natural COX-2 and immune activity and its manufacturing method, wherein the manufacturing method is easy and simply by making the Labiatae plant into a powder form.

Accordingly, in order to accomplish the above objects, the present invention provides a herbal composition having natural COX-2 and immune activity, which comprises powder of a Labiatae plant.

A method of manufacturing a herbal composition having natural COX-2 and immune activity, comprising the steps of:

(a) providing a Labiatae plant;

(b) dehydrating the Labiatae plant; and

(c) grinding the dried Labiatae plant into a powdered form.

said he herbal composition has an administrative form selected from capsule, powder, solution, and syrup.

These and other objectives, features, and advantages of the present invention will become apparent from the following detailed description, the accompanying drawings, and the appended claims.

**BRIEF DESCRIPTION OF THE DRAWINGS**

**FIGS. 1 and 2**   is a photograph of the new invention, "Guizhou Qizhen grass", showing the outlook of the whole plant.

**FIG. 3**   is a photograph of the invention showing the outlook of the whole plant with roots.

**FIG. 4**   is a photograph of the invention showing its upright extending stem and flowers.

**FIG. 5**   is a photograph of the invention showing the outlook of its flow- ers in purplish red color.

**FIG. 6**   is a flow diagram illustrating a method of manufacturing a herbal composition, having natural COX-2 and immune activity, by Guizhou Qizhen grass.

## DETAILED DESCRIPTION OF THE PREFERRED EMBODIMENT

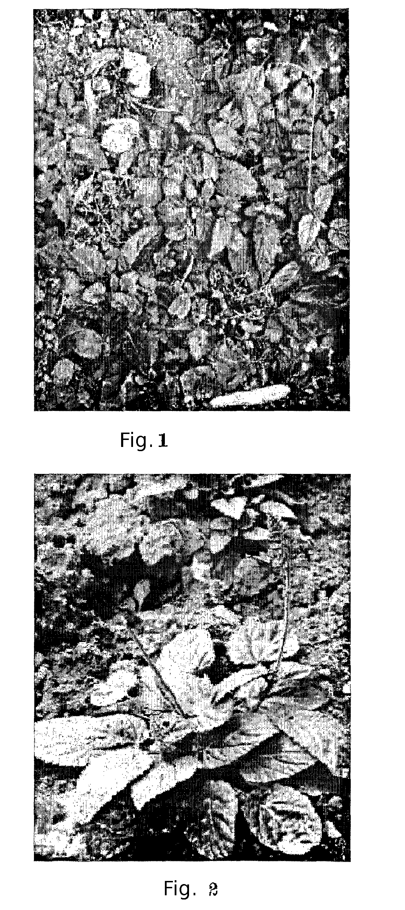

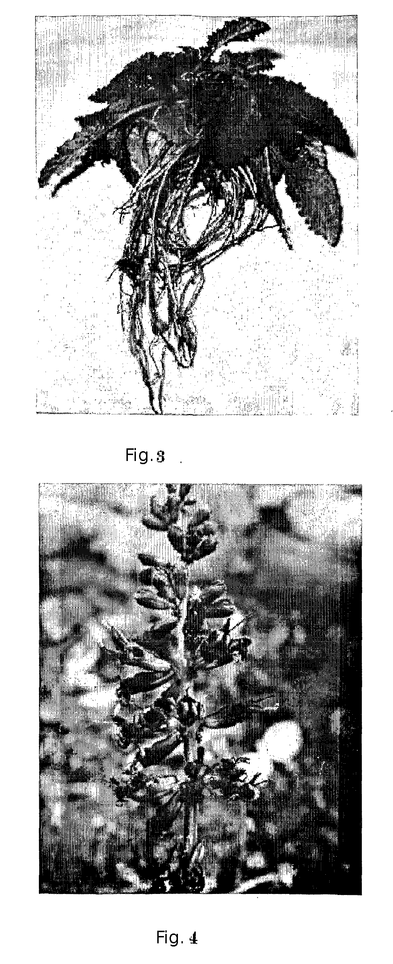

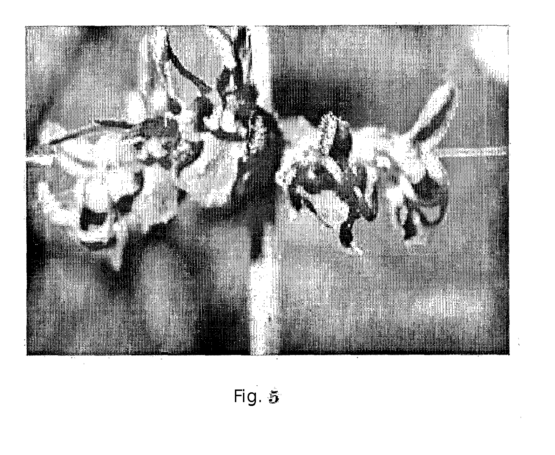

Referring to FIGS. 1 to 5, a herbal composition having natural COX-2 and immune activity and its manufacturing method are illustrated, wherein the herbal composition comprises a powder of Labiatae plant.

The new herb Guizhou Qizhen Grass discovered by the applicant differs from related herbal plants including Sallvia Multiorrhiza, Bge (Dan Shen), Scutellaria Indica L. (Han Sin Cao), Scutellaria Barbata Don (Ban Zhi Lian), and Scutellaria Discolor Colebr (Wa Er Cao), among which the Dan Shen has pulp roots and white flowers, the Wa Er Cao has a soft hairy stem, fibrous roots and blue flowers, and the Ban Zhi Lian is arranged like a toothbrush that has fibrous roots and blue flowers.

The herbal Labiatae Plant, which is a grass-like plant, known as "Guizhou Qizhen Cao". "Guizhou Qizhen Cao" is a distinctive variety of Labiatae plant, which is characterized by its quadri-angular stem, fibrous roots with beaded root nodules grown thereon, purplish red peduncles, and purplish red flowers.

The descriptions disclosed herein are based upon observations of the plant growth.

The size of the whole plant has a height of 10-30 cm. The stem which has a quadric-angular shape is an erect stem or a stolon stem. The roots are fibrous roots with beaded root nodules grown thereon. The leaves are in oval shape and grown from the roots. Each leaf has a heart shaped leaf base and a peripheral edge with shallow round teeth. Each plant has single leaf or 1-2 pairs of compound leaf. Each leaf has a leafstalk having a length of 8-12 cm. The two blade surfaces of each leaf are hairless and have plenty of wrinkles. The color of the leaves is green or purplish red. The plant has about 30 leaves at the stem portion while the leaves at the peduncle are in pair manner. The flowers are arranged in circular taper manner and there are 15-22 rounds of flowers, wherein each round has 6 flowers and each flower has a size of 8x3 mm. The flowers are lip style flowers, each having a tubular shape and two slits at the lower lip. Each flower has two stamens. The style of each flower is grown from the ovary. The flowers are arranged at the top portion of the stem to form a whole series of flowers. The corolla of each flower is in purplish red color. The nuts are small and each has an elliptical shape and a dark red color. The nut is matured within the calyx.

The plant grows from seedling propagation, and can be cultured and propagated in garden. The entire plant may be used in oriental medicine. The growing condition prefers windy, warm and wet environment. The rest period of the plant is winter that the plant stops to grow and to grow leaf temporally. The plant has a slight fragrant and a bitter taste. Each acre may grow more than 300-400 kg of fresh plants for two seasons per year. The plant may be cultured twice a year, respectively during the time from March to April and from September to October. Therefore, the plant gets mature between April and October. Preferably, the plant is seeded between May and June each year, wherein the time can then be set for next seeding after the harvest.

According to the analysis conclusion drawn by the National Center for Drug Screening in Shanghai, this plant is rich in human cyclooxygenase-2 (COX-2) which is effective in curing different kinds of human diseases. Below is the test principle and experiment result:

Objective of the Experiment:

The COX metabolizes arachidonic acid, and thereby generates prostagiandin and reactive oxygen species (ROS). COX2 is a key factor for the occurrence and development of inflammation, but COX1 mainly regulates and controls physiological function. COX2 selective inhibitor avoids disturbing physiological function during anti-inflammatory and thus is an ideal target spot, which makes the screening of its inhibitor thereof has obvious application prospect.

Principle of the Experiment:

The COX metabolizes arachidonic acid, and thereby generates prostagiandin and ROS. Measuring the ROS fluorescent light or PG22 generation, and thus determine enzymatic activity.

Result of the Experiment:
Inhibition ratio (%) of the samples against COX-2 COX-2 enzyme

| microg/ml | 10 |
|---|---|
| NC00127983 | 7.42 |

| microM | 0.1 | 1 | 10 |
|---|---|---|---|
| Celecoxib | 34.05 | 70.55 | 92.10 |

The plant has immune activity as well, and the following are the immune method and result of immune activity experiment carried out by "National Center for New Drug Screening":
Name of the model: breeder reaction and cytotoxicity experiment of T,B lymphocytes
Screening method:

under the stimulus of mitogens ConA and LPS, BALB/C mouse spleen lymphocytes undertake a series of changes in cellular morphology and metabolism, and transform into brood cells which subsequently differentiate and proliferated. In this experiment, 5ug/ml Con A is added to induce the T lymphocyte proliferation, and 100 ug/ml LPS is added for inducing the proliferation of B lymphocyte. 3H-TdR incorporation is used to estimate cell proliferation. Mitochondria dehydrogenase in living cells reduces MTT from yellow into blue formazan product, and the production of the formazan product is in direction protection to living cells. Subsequent to the dissolution of the organic solvent, OD value is read by microplate reader.

Assessment of the experiment result:

lymphocytes Proliferation index =[(CPM value minus control CPM value)/ CPM value]%;

the presence of negative sign before the percentage indicates that the sample has a inhibiting action agaisnt T,B cells; the absence of negative sign before the percentage indicates that the sample has a reinforcing function on T,B cells; judgment upon the cytotoxicity of lymphocyte: "yes" , "no"
under the condition that the cell is found nontoxic, the inhibiting/reinforcing

| Sample | Determined concentration | CD a.v. | SD | P value | cyto toxicity Yes /No | | T lymphocyte LPS stimulus CPM a.v. value | SD | T cell proliferation Comprehensive evaluation Enhancing/ inhibit ing ratio | B lymphocyte LPS stimulus CPM a.v. value | SD | B cell proliferation Comprehen sive evaluation Enhancin g/ inhibi ting ratio |
|---|---|---|---|---|---|---|---|---|---|---|---|---|
| | | | | | | N CON | 1569 | 173 | | 1587 | 52 | |
| | | 0.287 | 0.001 | | | N CON | 34014 | 2299 | | 7434 | 506 | |
| NC00127 983 | 1:4000 | 0.243 | 0.030 | 0.1203 | No | | 30884 | 13747 | -9% | 7381 | 877 | -1% |
| | 1:400 | 0.278 | 0.032 | 0.6593 | No | | 16357 | 13445 | -52% | 5753 | 220 | -23% |
| | 1:40 | 0.430 | 0.032 | 0.0166 | No | | 7754 | 5290 | -77% | 3272 | 917 | -56% |

function percentage above ± 15% upon T,B lymphocytes represent effective.

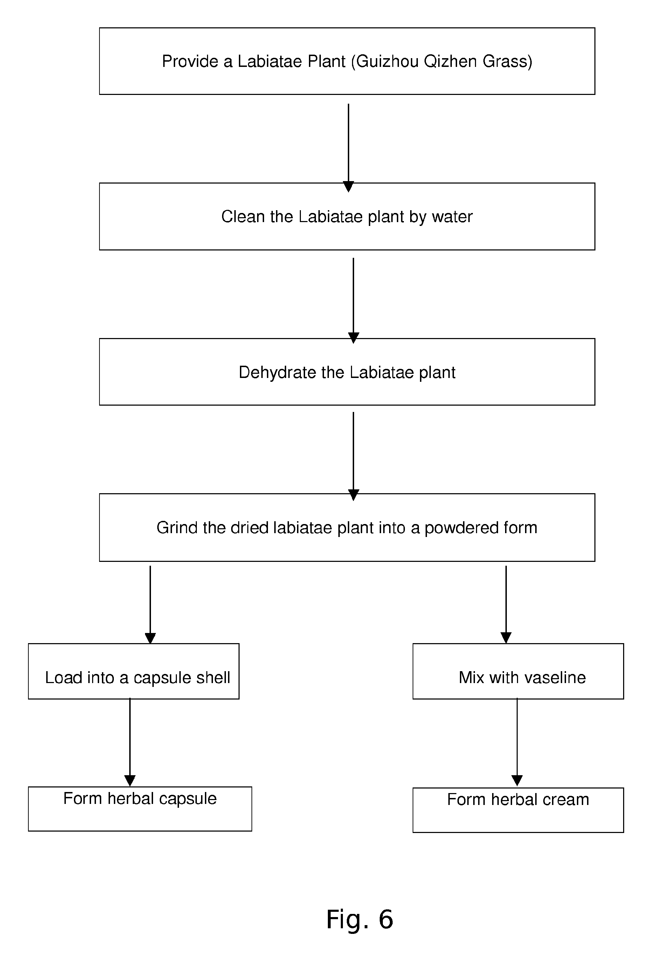

As shown in FIG. 6, the method of manufacturing the herbal composition having natural COX-2 and immune activity comprises the following steps.

(1) providing the whole Labiatae plant (Guizhou Qizhen grass).

(2) cleaning the Labiatae plant by water.

(3) dehydrating the Labiatae plant.

(4) grinding the dried Labiatae plant into a powdered form, preferably by grinding device.

According to the preferred embodiment, the herbal composition has an administrative form selected from the group consisting of capsule, powder, solution, and syrup.

In order for administration, the herbal composition further comprises a carrier loading with the powdered Labiatae plant, wherein the carrier comprises a capsule shell loading the powdered Labiatae plant therein to form a herbal capsule. The herbal capsule contains 0.35 g of the powdered Labiatae plant in the capsule shell.

The herbal composition is administrated in a dosage of 5.4 g per day for an adult for a treatment period ranging from 14 days to 42 days. In other words, the user will take the herbal composition three times a day and will take 1.8 g for each time. Therefore, the user will take five herbal capsules for each time. In particularly, the treatment period is set as seven days as one cycle. For acute inflammation, an adult will take two cycles, i.e. 14 days, as the treatment period. For chronic inflammation, an adult will take six cycles, i.e. 42 days, as the treatment period.

Alternatively, the carrier comprises "Vaseline" mixing with the powdered Labiatae plant to form a herbal cream as an external treatment to apply on an external injury area of a skin of a user, wherein a ratio of the powdered Labiatae plant and the "Vaseline" is 0.5:1 by weight.

Accordingly, the plant extract can rapidly subdue inflammation, swelling, and relieve pain. It can also eliminate viruses, inhibit toxic cells, activate the immune system, etc. After several years of observation on clinical application, the inventor discovered that Guizhou Qizhen grass has notable effects on curing acute and chronic nephritis, acute and chronic cholecystitis (including bile-reflux cholecystitis), pathological changes of lymphatic system like lymphatic tumors or submandibular gland tumors, gastric tumors, mastitis, and the recovery after polyp surgery of either human bodies or throat.

The ointment made of Guizhou Qizhen grass can cure sand holes and pinkeye; the external application can cure physical injury, burns from water or fire, sores and pus. If we stamp the original material and apply it to the body parts, it will help heal the fracture. If we add it to the Chinese herbal medicine and make it into facial ointment, it will beautify the skin through tightening, making the color lighter, removing wrinkles and brown or black freckles. By adopting Guizhou Qizhen grass, the above diseases will be cured rapidly and thoroughly without having a relapse, and the medicine even achieves 95% effectiveness. There are a lot more undiscovered applications; therefore, the inventor seeks to reserve the right for further study.

Below is brief introduction to some of the typical clinical cases:

I. Nephritis

1. Mr. Li. Guo Xiang, 65 years old, retired from Anshun in Guizhou. He had a nephrectomy and had his right kidney cut off. In October 2001, Mr. Li felt a pain in the left portion of his waist, and subsequently found occult blood, and then blood clot, mixed in his urine. During that time, Mr. Li visited several local hospitals, and even People's Liberation Army General Hospital in Beijing. All these hospitals requested the patient to take a cystoscope inspection, but the patient refused and gave up any further diagnosis and treatment. In May 2002, Mr. Li went to the inventor's place and tried the capsule made of Guizhou Qizhen grass. According to Mr. Li, there was not as much blood in his urine the day after he took the medicine, the swelling pain in his waist disappeared, and his urine went back to normal color on the third day. Mr. Li kept taking Guizhou Qizhen grass for two months, and five years has passed after that. During these 5 years, no relapse has occurred, and his body has been in a sound condition.

2. Mr. Wei, 66 years old, retired from Anshun railway station in Guizhou Province. He had suffered from chronic nephritis for several years, and his body has been in a very poor condition. At that time, he could only have about 50g of rice for each meal, and he always felt fatigued and drowsy. His legs had little strength, and he just stayed in bed except meal time. After taking Guizhou Qizhen grass for half of a month in 2001, Mr. Wei felt that his legs were getting the strength back, and his appetite was growing. After one month of medication, all his disease symptoms disappeared, and the nephrosis did not came back again.

3. Mr. Liu, 65 years old, retired from Anshun Xinhua bookstore in Guizhou. Mr. Liu started to feel bloated in his waist in his urine in June 2007, and then blood was viewed in his urine. The blood increased everyday. Being informed of the effectiveness of the medicine produced by the inventor, Mr. Liu turned to the inventor and tried Guizhou Qizhen grass. He told the inventor that the day after he took the medicine, "the blood in (my) urine was cut by two thirds", and he told the inventor on the third day that, "the urine become clear and there was no blood is viewed in it any more, and the swelling felling disappeared as well". Mr. Liu kept on taking the medicine for a month and had not relapsed.

4. Mrs. Liu, 60 years old, retired from an Anshun pharmaceutical company in Guizhou. In 2001, Mrs. Liu was admitted to a local hospital because of the following conditions: facial and foot dropsy, abdominal distention, and oliguria. Her symptoms were diagnosed as chronic nephritis with active break out, and four "positive marks" are exhibited in the urine test report. After consecutively injecting antibiotic medicine for more than 20 days, however, four "positive marks" remained in the urine test report. Mrs. Liu started to take Guizhou Qizhen grass after she left the hospital. In the afternoon of the very first day she started taking the medicament, her urine volume increased; she had normal amount of urine three days later. 7 days later, the dropsy in her face and feet disappeared seven days later, and she took another urine test, on the result of which the original four "positive marks" were turned to be negative ones. Mrs. Liu kept on taking the medicine successively for a month. 6 years has passed, no relapse has occurred. She now even has a better appetite and sleep than before.

5. Du Mei is a 24-year-old female from Guizhou Province and living in Zhejiang. In 2004, she started to feel bloated in her waist and found blood in her urine, and she felt fatigued and lost appetite. She visited local hospitals and experts in Chinese medicine from Shanghai, but had no improvement. She was referred to Anshun (where the inventor is located) by her relatives in 2005. Du Mei had a urine test before the treatment and "positive marks" were shown in her occult-blood report, four "positive marks" in her corpuscle report, and three "positive marks" were shown in her urine-protein report. She was very weak when she first visited the inventor. According to Du Mei, her feet and legs had little strength, she lost appetite, and she had medium foot edema which is a typical syndrome of chronic nephritis. The day after she took Guizhou Qizhen grass, she said that there was less blood in her urine and she did not feel as bloated in her waist. Du Mei kept taking the medicine. On the third day, her foot edema was less serious, she could urinate more and the color of her urine turned to be lighter; she felt more energetic and she had more strength to walk. After taking the medicine for 12 days, Du Mei went to have another urine test. This time, the report showed that no blood is found in her urine, her white corpuscles and urine protein came to be normal. Du Mei continued taking the medicine for another two months, and no relapse occurred. Now, she is in a very sound condition.

II. Lymphoma and Colorectal Tumor

1. Mrs. Xiao, 64 years old, retired from an Anshun local governmental institute in Guizhou. In 1998, Mrs. Xiao found two lumps underneath her upper jaw confirmed by a doctor as submandibular gland tumors with a radius of $36.5 \times 16$ mm. Afterward, Mrs. Xiao visited several hospitals and tried all kinds of medicine, but without improvement. The lumps grew from $36.5 \times 16$ mm to 45.6 mm, and pain began to be felt. Initially, Mrs. Xiao planned to have surgery, but she turned to try treatment with Guizhou Qizhen grass instead, upon the recommendation of another patient. In less than 2 months, the lumps became smaller, from 45.6 mm to $13.9 \times 9.1$ mm and then 9x10 mm, and disappeared after Mrs. Xiao completed one month of treatment. No relapse occurred, and her body has kept to be in a very sound condition.

2. Mrs. Jiang, 40 years old, worked in Anshun train station in Guizhou. In 1996, Mrs. Jiang felt a lump in her neck, and she went to a hospital wherein it's confirmed that she had a "chronic inflammation of neck organization" with "a lot of lymphatic tissue soaking." Mrs. Jiang extremely worried about her disease, so she visited the province hospital and tried all kinds of medicine, but no improvement is achieved. Her lump grew as big as a chestnut, turned to be painful. What's worse, granular lumps of different sizes appeared in the soft tissue of her joints, and the tissue of her nasal cavity grew bigger and harder with irregular bleeding. Her family worried about her very much. Mrs. turned to the inventor and tried Guizhou Qizhen grass. Several days later, her nasal cavity stopped bleeding, the tissues in her nasal cavity became softer and the lumps disappeared after two months. Mrs. Jiang said that there was only a tiny lump in her neck by that time. She continued taking the medicine for another month, and she has been in good condition without any relapses.

3. Mr. Mei, 63 years old. In 1998, he started to have trouble in defecating, and the blood jetted out while defecating. After a anal examination in hospital, he was confirmed to have a lump as big as a chestnut, located 15 cm from his anus. No improvement is achieved after the medical treatment and the lump kept growing. The excrement of the patient, as a result, became as thin as a pencil accompanied by blooding. Mr. Mei was very sick at that time. He started to take Guizhou Qizhen grass. On the third day, hematochezia is stopped, and it became a lot easier to defecate after half of a month; the size of the lump, according to Mr. Mei, became smaller. He continued to take the medicine for another month, and his body now is healthier than before.

III. Acute, Chronic, and Bile-Reflux Cholecystitis

1. Mrs. Xu, 65 years old, retired from an Anshun Knitting Factory in Guizhou Province. She had suffered from chronic cholecystitis for many years, and it broke out easily in case of any slightest carelessness for her food, and the pain accompanying the attack is unbearable. As a result, she could only have light food and she always worried about the pain. In 1999, her son bought Guizhou Qizhen grass to her. She kept taking the medicine for a month, and then the inflammation is gone, and no relapse occurred. She does not worry about her meals anymore and has been in good health.

2. Mrs. Yu, 62 years old, is from Pingba in Guizhou Province. She had suffered from bile-reflux cholecystitis for many years. She could not have any poultry eggs, meat, or any other greasy food; the disease broke out often and the accompanying pain was unbearable. In 2003, her daughter brought her Guizhou Qizhen grass. After only one month of taking this medicine, Mrs. Yu needs not worry about the choice of food anymore, and has no relapse.

3. Mrs. Zhang, 50 years old, works in light industry in Guizhou Province. She had suffered from bile-reflux cholecystitis for several years, and her situation is so severe that any words mentioning feast will cause her vomit. She used to have only half bowl of porridge and was very sick. In 2002, she took Guizhou Qizhen grass for two months, and now she is in good condition. Besides, Mrs. Zhang's daughter also had congenital cholecystitis, but recovered after taking the medicine for two months and has no relapse.

4. Mrs. Chen, 52 years old, worked for Anshun Pharmaceutical Company in Guizhou Province. She had suffered from lithiasis cholecystitis, coronary heart disease, hypertension, diabetes, and so on. Due to the frequent attack of cholecystitis, Mrs. Chen had no choice but to be a vegetarian. As a result, her body was in a under-nurishment condition and had a weak resistance against diseases, and she even had trouble going outside of the house. In 2003, she began to take Guizhou Qizhen grass, and the inflammation disappeared after half a month and her appetite came back. Mrs. After a month of taking this medicine, Chen gradually recovered and she could go traveling. Her sound body condition persists.

IV. Aphonia after Throat Polyp Surgery

1. Mrs. Cai, 45 years old, is a manager of a construction company in Guizhou Province. In 2005, she had a laser treatment for the polyp in her throat. After the treatment, she suffered from severe dry sensation in her throat, which soon caused aphonia. Mrs. Cai tried all kinds of medical treatment, but no curative effect is achieved. In a chance, her brother got Guizhou Qizhen grass from the inventor and brought the same to the patient. In the next day after she took the medicine, she got her voice back and could speak as usual, but aphonia often came back as soon as she stopped taking the medicine. Understanding this situation, Ms. Cai insisted on the taking of this medicine for successive two month, after which she recovered and got her normal voice back.

2. Ms. Wen, 26 years old, is a teacher in an Anshun No. 1 Kindergarten in Guizhou Province. In 1999, Ms. Wen accepted a laser treatment for polyp in her throat. She suffered from severe aphonia for a long time after she left the hospital. After she took Guizhou Qizhen Grass for a month, the discomfort in her throat disappeared and she had her voice back. There were no relapses until now.

In 2006, Guizhou Qizhen grass was confirmed by the National Center for Drug Screening in China that it has natural COX-2 activity and immunoregulating activity which will cure inflammation. Meanwhile, the plant can control lymphatic cells T and B, so it has strong potential that need to be discovered.

The medicine is made of 1.8 grams per dose. A patient should take 5.4 grams of water-soluble medicine per day. The prescription for different disease works best when: the patient having acute nephritis and cholecystitis should take two treatment cycles (fourteen days), wherein each treatment cycle is seven days. The patient having chronic nephritis should take four treatment cycles, i.e. twenty-eight days. The patient having tumor should take eighteen treatment cycles, i.e. one hundred and fifty-six days. The patient having aphonia should take three treatment cycles, i.e. twenty-one days and there is no relapse. The medicine is prepared by the extracted Guizhou Qizhen grass and formed with petroleum jelly ("Vaseline") for curing the eyes or external injury. The ratio of the medicine to petroleum jelly is 0.5: 1 by weight.

It will thus be seen that the objects of the present invention have been fully and effectively accomplished. One skilled in the art will understand that the embodiment of the present invention as shown in the drawings and described above is exemplary only and not intended to be limiting. Therefore, this invention includes all modifications encompassed within the spirit and scope of the following claims.

**Claims**

1. A method of manufacturing a herbal composition having natural COX-2 and immune activity, comprising the steps of:

   (a) providing a Labiatae plant (Guizhou Qizhen grass);
   (b) dehydrating said Labiatae plant; and
   (c) grinding said dried Labiatae plant into a powdered form.

2. The method according to claim 1 wherein, before the step (b), said Labiatae plant is cleaned and washed by water.

3. The method according to claim 2, further comprising a step of (d) loading said powdered Labiatae plant into a capsule shell to form a herbal capsule.

4. The method according to claim 3, wherein said herbal capsule contains 0.35 g of said powdered Labiatae plant in said capsule shell.

5. The method according to claim 2, further comprising a step of (d) mixing said powdered Labiatae plant with "Vaseline" to form a herbal cream.

6. The method according to claim 5, wherein a ratio of said powdered Labiatae plant and said "Vaseline" is 0.5:1 by weight.

7. A herbal composition having natural COX-2 and immune activity, comprising a powder of Labiatae plant (Guizhou Qizhen grass), wherein said herbal composition is prepared by a method comprising the steps of:

   (a) providing said Labiatae plant;
   (b) dehydrating said Labiatae plant; and
   (c) grinding said dried Labiatae plant into a powdered form.

8. The herbal composition according to claim 7, further comprising a carrier loading with said powdered Labiatae plant, wherein said carrier comprises a capsule shell loading said powdered Labiatae plant therein to form a herbal capsule.

9. The herbal composition according to claim 8, wherein said herbal capsule contains 0.35 g of said powdered Labiatae plant in said capsule shell.

10. The herbal composition according to claim 7, further comprising a carrier loading with said powdered Labiatae plant, wherein said carrier comprises "Vaseline" mixing with said powdered Labiatae plant to form a herbal cream.

11. The herbal composition according to claim 10, wherein a ratio of said powdered Labiatae plant and said "Vaseline" is 0.5:1 by weight.

12. The herbal composition according to claim 7, having an administrative form selected from the group consisting of capsule, powder, solution, and syrup.

13. The herbal composition according to claim 7, which is administrated in a dosage of 5.4 g per day for an adult for a treatment period ranging from 14 days to 42 days.

**14.** The herbal composition according to claim 7, which is administrated in a dosage of 5.4 g per day for an adult for acute inflammation with a treatment period of 14 days.

**15.** The herbal composition according to claim 7, which is administrated in a dosage of 5.4 g per day for an adult for chronic inflammation with a treatment period of 42 days.

**16.** A method for the treatment of a disease treated with natural COX-2 and immune activity, comprising a step of administrating an effective amount of herbal composition containing Labiatae plant (Guizhou Qizhen grass), wherein said herbal composition is prepared by a method comprising the steps of:

    (b) dehydrating said Labiatae plant; and
    (c) grinding said dried Labiatae plant into a powdered form.

**17.** The method according to claim 16, wherein said disease is selected from the group consisting of Nephritis, Lymphoma and colorectal tumor, Acute, chronic, and bile-reflux cholecystitis, and aphonia.

**18.** The method according to 16, wherein said Labiatae plant is administrated in form of capsule by loading a powder form of said Labiatea plane into a capsule shell.

**19.** The method according to claim 16, wherein said Labiatae plant is administrated in form of cream by mixing said powdered Labiatae plant with "Vaseline" to apply on an external injury area of a skin of a user.

**20.** The method according to claim 16, wherein said Labiatae plant is administrated in form of capsule by mixing a powder form of said Labiatea plane with water.

Fig. 1

Fig. 2

Fig. 3

Fig. 4

Fig. 5

Provide a Labiatae Plant (Guizhou Qizhen Grass)

Clean the Labiatae plant by water

Dehydrate the Labiatae plant

Grind the dried labiatae plant into a powdered form

Load into a capsule shell

Mix with vaseline

Form herbal capsule

Form herbal cream

Fig. 6

# INTERNATIONAL SEARCH REPORT

| International application No. |
|---|
| PCT/CN2009/000134 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC   A61K,A61P

Documentation searched other than minimum documentation to the extent that such documents are included in the fields searched

Electronic data base consulted during the international search (name of data base and, where practicable, search terms used)

WPI, EPODOC, PAJ, CNKI, CNPAT(Labiatae,salvia miltiorrhiza,scutellaria
barbata,cyclooxygenase,immune, COX, GUIZHOUQIZHENCAO, inflammation,nephritis,cholecystitis,tumor,polyps)

**C. DOCUMENTS CONSIDERED TO BE RELEVANT**

| Category* | Citation of document, with indication, where appropriate, of the relevant passages | Relevant to claim No. |
|---|---|---|
| X | CN1961763A(YAN Guang),16 May 2007(16.05.2007),abstract | 1-3,16-18,20 |
| X | WO2007084419A2(FEINSTEIN INST MEDICAL RES),26 Jul.2007(26.07.2007),abstract | 14-15,19 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
|---|---|
| 26 Oct.2009(26.10.2009) | **19 Nov. 2009 (19.11.2009)** |

| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br><br>ZHAI, Yu<br><br>Telephone No. (86-10)62411066 |
|---|---|

Form PCT/ISA /210 (second sheet) (July 2009)

# INTERNATIONAL SEARCH REPORT

International application No.

PCT/CN2009/000134

**Box No. II    Observations where certain claims were found unsearchable (Continuation of item 2 of first sheet)**

This international search report has not been established in respect of certain claims under Article 17(2)(a) for the following reasons:

1. ☒  Claims Nos.: 16-20
     because they relate to subject matter not required to be searched by this Authority, namely:
     The method for treating diseases using the herbal composition having natural and immune biological activities as claimed in claims 16-20 is related to treatment of disorders of human and animals, therefor, they belong to subject matters not required to be searched. So claims 16-20 are based on assumptional claims, i.e. the use of a herbal composition as described in any one of claims 1-15 for preparation of a medicament having natural COX-2 and immune biological activities.

2. ☐  Claims Nos.:
     because they relate to parts of the international application that do not comply with the prescribed requirements to such an extent that no meaningful international search can be carried out, specifically:

3. ☐  Claims Nos.:
     because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying an additional fees, this Authority did not invite payment of additional fee.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on protest**        ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA /210 (continuation of first sheet (2)) (July 2009)

## INTERNATIONAL SEARCH REPORT
Information on patent family members

| | International application No. |
|---|---|
| | PCT/CN2009/000134 |

| Patent Documents referred in the Report | Publication Date | Patent Family | Publication Date |
|---|---|---|---|
| CN1961763A | 16 May 2007(16.05.2007) | none | |
| WO2007084419A2 | 26 Jul.2007(26.07.2007) | EP1981489A2 | 22 Oct.2008(22.10.2008) |
| | | AU2007207675A1 | 26 Jul. 2007(26.07.2007) |

Form PCT/ISA /210 (patent family annex) (July 2009)

# INTERNATIONAL SEARCH REPORT

International application No.

PCT/CN2009/000134

CLASSIFICATION OF SUBJECT MATTER

A61K36/53(2006.01)i

A61K9/06(2006.01)i

A61K9/08(2006.01)i

A61K9/14(2006.01)i

A61K9/48(2006.01)i

A61P1/16(2006.01)i

A61P13/12(2006.01)i

A61P29/00(2006.01)i

A61P37/00(2006.01)i

 A61P37/02(2006.01)i

A61K125/00(2006.01)n

Form PCT/ISA /210 (extra sheet) (July 2009)